# EUROPEAN PATENT APPLICATION

(11) **EP 1 674 100 A1**
(43) Date of publication of application: **28.06.2006**
(21) Application number: 04792224.0
(22) Date of filing: 08.10.2004
(51) Int. Cl.: A61K 31/444, A61P 35/00, A61P 27/02, A61P 27/06, A61P 3/10, C07D 401/14

(54) **ANGIOGENESIS INHIBITOR**

(30) Priority: 10.10.2003 US 510012 P
(71) Applicant: Kowa Co., Ltd., Naka-ku, Nagoya-shi, Aichi 460-8625 (JP)
(72) Inventor: DOI, Takeshi, Higashiyamoto-shi, Tokyo 207-0011 (JP); TAMURA, Masahiro, Machida-Shi, Tokyo 1940003 (JP)
(74) Representative: Hartz, Nikolai
(86) International application number: PCT/JP2004/014956
(87) International publication number: WO 2005/034953

(57) **Abstract**

The present invention relates to a prophylactic or therapeutic agent against a disease or pathological condition caused by angiogenesis, comprising, as an active ingredient, a cyclic amine compound represented by the general formula (1): 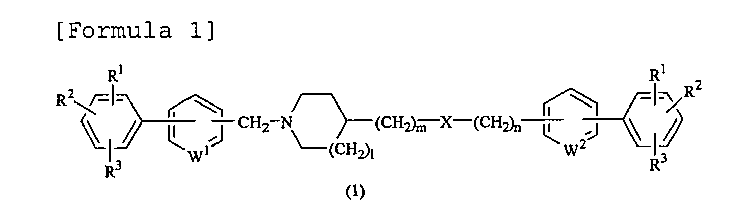 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, -or NR⁹-CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and l, m, and n each represent a number of 0 or 1, or a salt thereof, or a solvate thereof.

## Description

### Technical Field

The present invention relates to an angiogenesis inhibitor. Specifically, the present invention relates to a prophylactic or therapeutic agent against a disease or condition in which angiogenesis is involved, such as, for example, proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

### Background Art

Angiogenesis refers to a phenomenon that new vessels are formed from existing vessels, and is known to be physiologically essential e.g. for wound healing and placentation during pregnancy. Meanwhile, angiogenesis has been also indicated to be responsible for the formation of a disease or pathological condition (Carmeliet P., Nature Med., 9(6), 653-660 (2003)). In addition to proliferation, recurrence, or metastasis of malignant solid tumor, a disease or condition particularly known in ophthalmologic field, such as corneal angiogenesis, pterygium, conjunctivitis, rubeosis iridis, neovascular glaucoma, proliferative retinopathy, centralretinalvein occlusion,diabetic retinopathy, retinal angiogenesis, or age-related macular degeneration is known as a disease or pathological condition mentioned above. (Chang JH, Gabison EE, Kato T, Azar DT, Curr. Opin. Ophthalmol. , 12 (4), 242-249 (2001)).
As a mechanism of angiogenesis, a process is known from the activation of vascular endothelial cells by factors such as VEGF (vascular endothelial growth factor) and FGF (fibroblast growth factor)-2 to the digestion of vascular basement membranes by proteases such as matrix metalloprotease and urokinase, and to the subsequent proliferation/migration and redifferentiation (luminal formation and basement membrane regeneration) of the cells and the reconstruction of vascular walls by perithelial cells.

In view of such a background, attention has been given in recent years to development of a therapeutic agent using angiogenesis inhibition as a mechanism, and studies of angiogenesis inhibitors based on various action mechanisms including inhibited VEGF function have been widely carried out (Chang JH, Gabison EE, Kato T, Azar DT, Curr. Opin. Ophthalmol., 12(4), 242-249 (2001) and Dredge K, Dalgleish AG, Marriott JB, Curr. Opin. Investig. Drugs, 4(6), 667-674 (2003)). However, it is yet hard to say that sufficient outcomes have been achieved, and there is need for development of a new agent having an angiogenesis-inhibiting effect.

### Disclosure of the Invention

Thus, an object of the present invention is to provide a novel angiogenesis inhibitor, and a prophylactic or therapeutic agent, using the same, against a disease or condition in which angiogenesis is involved, for example, proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

In view of the current condition, as the result of searching for substances having an effect on angiogenesis using cultured cell lines, the present inventors have found that compounds represented by the general formula (1) to be described have, very surprisingly, an excellent angiogenesis-inhibiting effect and are useful as a prophylactic or therapeutic agent against proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, age-related macular degeneration, and the like, thereby accomplishing the present invention.

Thus, the present inventionprovides a method for treating a disease or condition caused by angiogenesis, comprising administering an effective amount of a cyclic amine compound represented by the general formula (1), a salt or a solvate thereof:

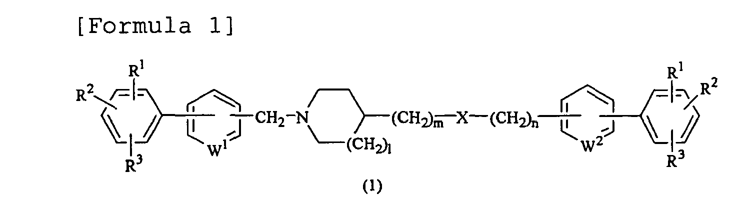

wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1.
The present invention also provides a method for inhibiting angiogenesis, comprising administering an effective amount of a cyclic amine compound represented by the formula (1) above, or a salt thereof, or a solvate thereof to patients in need thereof.

The present invention further provides an angiogenesis inhibitor comprising, as an active ingredient, a cyclic amine compound represented by the formula (1) above, or a salt thereof, or a solvate thereof.
The present invention further provides a prophylactic or therapeutic agent against a disease or condition caused by angiogenesis, comprising, as an active ingredient, a cyclic amine compound represented by the formula (1) above, or a salt thereof, or a solvate thereof.

The present invention further provides use of a cyclic amine compound represented by the formula (1) above, or a salt thereof, or a solvate thereof for producing an angiogenesis inhibitor.
The present invention further provides use of a cyclic amine compound represented by the formula (1) above, or a salt thereof, or a solvate thereof for producing a prophylactic or therapeutic agent against a disease or condition caused by angiogenesis.

According to the present invention, a novel angiogenesis inhibitor can be provided, and a prophylactic or therapeutic agent can be further provided against a disease or condition in which angiogenesis is involved, such as proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

### Brief Description of the Drawings

Figure 1 is a picture showing the condition of angiogenesis when each of the compounds was added; and
Figure 2 is a pair of graphs showing effects of each of the compounds on tubule area (A) and tubule length (B).

### Best Mode for Carrying Out the Invention

The compounds represented by the general formula (1) used in the present invention have an excellent cell adhesion-inhibiting effect as described in WO03/02703, which discloses the application thereof to allergic disease, autoimmune disease, and chronic inflammatory disease, but itisundisclosed whethertheyhave an angiogenesis-inhibiting effect or not. The content of WO03/02703 including a process for producing compounds of the general formula (1) and a method for preparing formulations containing compounds of the general formula (1) is herein incorporated by reference.

In the formula (1), halogen atoms represented by R¹ to R³ include a fluorine atom, a chlorine atom, a bromine atom, and an iodine atom.

Alkyl groups represented by R¹ to R⁴ include C₁-C₈ linear, branched, or cyclic alkyl groups; the C₁-C₈ linear or branched alkyl groups include, for example, methyl, ethyl, propyl, butyl, pentyl, hexyl, heptyl, and octyl, and the C₃-C₈ cyclic alkyl groups include, for example, cyclopropyl, cyclobutyl, cyclopentyl, cyclohexyl, cyclohexylmethyl, and cyclohexylethyl. Among these, C₁-C₆ alkyl groups such as methyl, ethyl, n-propyl, isopropyl, and n-butyl are particularly preferable.

Halogen-substituted alkyl groups represented by R¹ to R³ include C₁-C₈ alkyl groups each substituted by 1 to 3 halogen atoms; among these, C₁-C₆ alkyl groups each substituted by 1 to 3 halogen atoms, such as trifluoromethyl and 2,2,2-trifluoroethyl are particularly preferable.

Alkoxy groups include C₁-C₈ linear, branched, or cyclic alkoxy groups; the C₁-C₈ linear or branched alkoxy groups include, for example, methoxy, ethoxy, n-propoxy, isopropoxy, n-butoxy, isobutoxy, sec-butoxy, tert-butoxy, pentyloxy, and hexyloxy, and the C₃-C₈ cycloalkyloxy groups include, for example, cyclopropyloxy, cyclobutyloxy, cyclopentyloxy, cyclohexyloxy, cyclohexylmethyloxy, and cyclohexylethyloxy. Among these, C₁-C₆ alkoxy groups such as methoxy, ethoxy, n-propoxy, isopropoxy, and n-butoxy are particularly preferable.

Alkylthio groups include C₁-C₈ alkylthio groups; for example, C₁-C₆ alkylthio groups such as methylthio, ethylthio, n-propylthio, and isopropylthio are preferable.

Alkoxycarbonyl groups include C₁-C₆ alkoxycarbonyl groups; for example, C₁-C₄ alkoxycarbonyl groups such as methoxycarbonyl, ethoxycarbonyl, and tert-butoxycarbonyl are preferable.

Alkanoyl groups include C₁-C₆ alkanoyl groups; for example, C₁-C₄ alkanoyl groups such as acetyl, propionyl, butylyl, and isobutylyl are preferable.

Alkenyl groups represented by R⁴ include C₃-C₈ alkenyl groups; for example, C₃-C₆ alkenyl groups such as 2-propenyl, and 3-butenyl are preferable. Alkynyl groups include C₃-C₈ alkynyl groups; for example, C₃-C₆ alkynyl groups such as 2-propynyl and 3-butynyl are preferable.

Aryl groups represented by R⁴ include C₆-C₁₄ aryl groups; for example, phenyl, naphtyl, anthryl, indenyl, indanyl, and 5,6,7,8-tetrahydronaphthyl are preferable.

Heteroaryl groups represented by R⁴ include 5- or 6-membered heteroaryl groups containing 1 to 4 nitrogen atoms; for example, imidazolyl, pyridyl, and pyrimidinyl are preferable. Aralkyl groups represented by R⁴ include C₆-C₁₄ aryl-C₁-C₆ alkyl groups, including, for example, phenyl-C₁-C₆ alkyl or naphtyl-C₁-C₆ alkyl groups such as benzyl, naphtylmethyl, phenylethyl, and phenylpropyl. Heteroaralkyl groups represented by R⁴ include 5- or 6-membered heteroaryl-C₁-C₆ alkyl group containing 1 to 4 nitrogen atoms, including, for example, imidazolyl-C₁-C₆ alkyl groups, pyridyl-C₁-C₆ alkyl groups, and pyrimidinyl-C₁-C₆ alkyl groups.

Groups with which these aryl, heteroaryl, aralkyl, or heteroaralkyl groups can be substituted may be 1 to 3 groups or atoms selected from an alkyl group, an alkoxy group, a halogen-substituted alkoxy group, an alkylthio group, an alkylsulfinyl group, an alkylsulfonyl group, a halogen atom, a nitro group, an amino group, an acetylamino group, a trifluoromethyl group, and an alkylenedioxy group. Here, alkyl, alkoxy and alkylthio groups include the same as those described in the above R¹ to R³. The alkyl groups of alkylsulfinyl and alkylsulfonyl groups include C₁-C₃ alkyl groups, particularly methyl, ethyl, n-propyl, and isopropyl. The halogen-substituted alkoxy group is preferably a C₁-C₈ alkoxy group substituted by 1 to 3 halogen atoms, particularly a C₁-C₄ alkoxy group substituted by 1 to 3 halogen atoms, including trifluoromethoxy or 2,2,2-trifluoroethoxy. Alkylenedioxy groups include C₁-C₃ alkylenedioxy groups such as methylenedioxy, ethylenedioxy, and propylenedioxy.

X is preferably NR⁴ where R⁴ is preferably a C₁-C₈ alkyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

Preferably, R¹, R² and R³ bond to the 3-, 4-, and 5-positions of the phenyl group, respectively. Here, it is particularly preferred that R¹ and R³ (that is, the 3- and 5-positions on the phenyl group) are an alkoxy group or a halogen atom. R² (that is, the 4-position on the phenyl group) is preferably a hydrogen atom, a halogen atom, a hydroxy group, an alkyl group, a halogen-substituted alkyl group, an alkoxy group, an alkylthio group, a carboxy group, an alkoxycarbonyl group, or an alkanoyl group.

Although 1 represents 0 or 1, 1 is preferable.
W¹ is preferably N. W² is also preferably N.

Among the compounds of formula (1), those where X is NR⁴ and R⁴ is a C₁-C₈ alkyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms are preferable. Further, it is particularly preferable that R⁴ is a phenyl or pyridyl group optionally substituted by one or two groups or atoms selected from a halogen atom, an alkyl group, an alkoxy group, an alkylthio group, a trifluoromethyl group, and an alkylenedioxy group. Alternatively, a C₁-C₈ alkyl group is particularly preferable.

More specifically, 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof is most preferable.

Salts of the above compound (1) are not particularly restricted in so far as they are pharmaceutically acceptable salts, but can include, for example, mineral acid addition salts such as hydrochloride, hydrobromate, hydroiodide, sulfate, and phosphate; and organic acid addition salts such as benzoate, methanesulfonate, ethanesulfonate, benzenesulfonate, p-toluenesulfonate, oxalate, maleate, fumarate, tartrate, citrate, and acetate.

In addition, the above-described compound (1) can be present in the form of a solvate typified by a hydrate, and the solvate is also intended to be embraced in the present invention.

The compound (1) has an effect of potently inhibiting angiogenesis as shown in Example to be described, and is useful as a prophylactic or therapeutic agent against a disease or pathological condition caused by angiogenesis in mammals including human, such as, for example, proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

The medicine according to the present invention contains the above-described compound (1), a salt thereof, or a solvate thereof as an active ingredient, and the mode of administration is not particularly restricted and may be properly selected depending on the therapeutic purpose; examples thereof include oral administration using tablets, capsules, granules, powders, syrups, or the like and parenteral administration using intravascular injections, intramuscular injections, suppositories, ointments, inhalants, eye drops, nasal drops, patches or the like. Compositions suitable for these modes of administration are formulated with pharmaceutically acceptable carriers, and may be produced by a conventional preparation method well known to those skilled in the art. The carrier may use the proper combination of excipient, binder, extender, disintegrator, surfactant, lubricant, dispersant, buffer, preservative, coloring agent, flavor, smell correcting agent, perfume, film forming agent, diluent, and the like.

The dosage of the medicine according to the present invention varies depending on the age, sex, body weight, and symptoms, the mode and frequency of administration, and the like; however, it is preferable that the medicine is usually administered orally or parenterally at a daily dose of 0.01 to 1000 mg, preferably 0.1 to 100 mg in the above-described compound (1) once or in several divided portions.

### Examples

The present invention is further specifically described below, based on Examples. However, the scope of the present invention is not intended to be limited by these Examples.

### Synthesis Example 1

Synthesis of 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine citrate
The synthesis was carried out according to Example 13 (4-[N-(4-methoxyphenyl)-N-[[5-(3,4,5-trimethoxyphenyl)py ridin-3-yl]methyl]amino]-1-[[2-(3,4,5-trimethoxyphenyl)p yridin-4-yl]methyl]piperidine trihydrochloride) described in WO03/02703, followed by converting the resultant free base to the citrate thereof to provide the title compound in the form of white to pale yellow powder.

Decomposition point: 126 to 129°C
NMR: (400MHz, DMSO-d₆) δ: 1.66 to 1.75 (m, 2H), 1.84 (d, 2H, J=10.7 Hz), 2.30 (t, 2H, J=11.5 Hz), 2.68 (dd, 4H, J=3.7 Hz, 15.4 Hz), 2.98 (d, 2H, J=11.2 Hz), 3.50 to 3.55 (m, 1H), 3.64 (s, 3H), 3.69 (s, 2H), 3,71 (s, 3H), 3.73 (s, 3H), 3.84 (s, 6H), 3.86 (s, 6H), 4.46 (s, 2H), 6.76 (d, 2H, J=9.0 Hz), 6.85 (s, 2H), 6.86 (d, 2H, J=8.8 Hz), 7.83 (d, 1H, J=4.9 Hz), 7,35 (s, 2H), 7.83 (s, 2H), 8.42 (s, 1H), 8.57 (d, 1H, J=4.9 Hz), 8.68 (d, 1 H, J=2.2 Hz)

### Example 1: Examination of an angiogenesis-inhibiting effect using cultured cells

As an in vitro angiogenesis model, a model for evaluating tubule formation induced by coculturing vascular endothelial cells and fibroblast has been reported (Bishop ET, Bell GT, Bloor S, Broom IJ, Hendry NFK, Wheatley DN, Angiogenesis, 3, 335-344 (1999)). This system was used to examine the effects of compounds on angiogenesis.

### 1) Compounds used

In addition to the compound obtained in Synthesis Example 1 above (hereinafter denoted as compound 1), a compound described in Example 10 of WO03/02703, i.e., 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine trihydrochloride (hereinafter referred to as compound 2) synthesized according to the described method was used for the following examination.

### 2) Preparation of compound solutions

Compound 1 and 2 were each dissolved in dimethylsulfoxide (DMSO) to prepare 3, 1, 0.3, and 0.1 mmol/L of compound solutions. Each of the DMSO solutions of the compounds (1 and 2) with the concentrations (0.1 to 3 mmol/L) prepared, or DMSO (for a compound-free group) was diluted 1, 000 times using a medium dedicated to angiogenesis (from Kurabo Industries Ltd.) containing 10 ng/mL VEGF-A (from Kurabo Industries Ltd.), to prepare 0 (compound-free group), 0.1, 0.3, 1, or 3 µmol/L of a solution of each of the compounds (1 and 2). The medium dedicated to angiogenesis containing DMSO, to which VEGF-A was not added, was also prepared as a negative control.

### 3) Examination of an angiogenesis-inhibiting effect of the compounds

### A. Test method

The angiogenesis-inhibiting effects of the compounds were evaluated using "Angiogenesis Kit" from Kurabo Industries Ltd. according to the procedure described in the protocol thereof. After obtaining the cells, the cell culture medium was exchanged every 2 to 3 days, and immunostaining was performed using an anti-CD 31 antibody on the 11th day of culture to observe and photograph the microvessel-like structure formed under a microscope. The resultant image data was analyzed using an image processing/quantitation software (from Kurabo Industries Ltd.) to determine the activity of inhibiting angiogenesis.

### B. Results

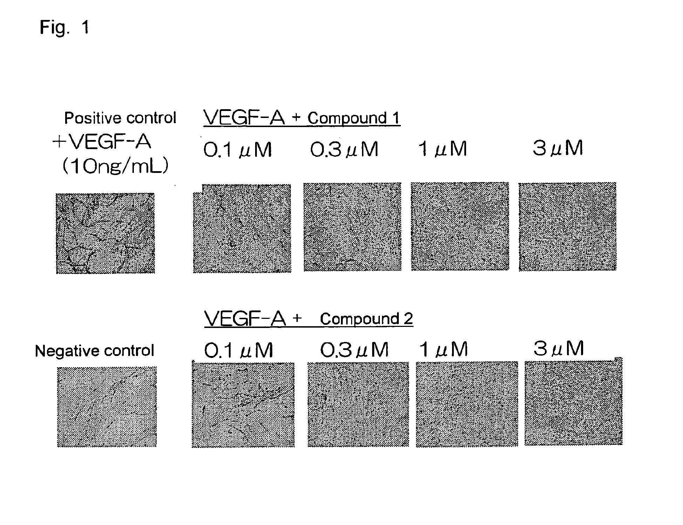

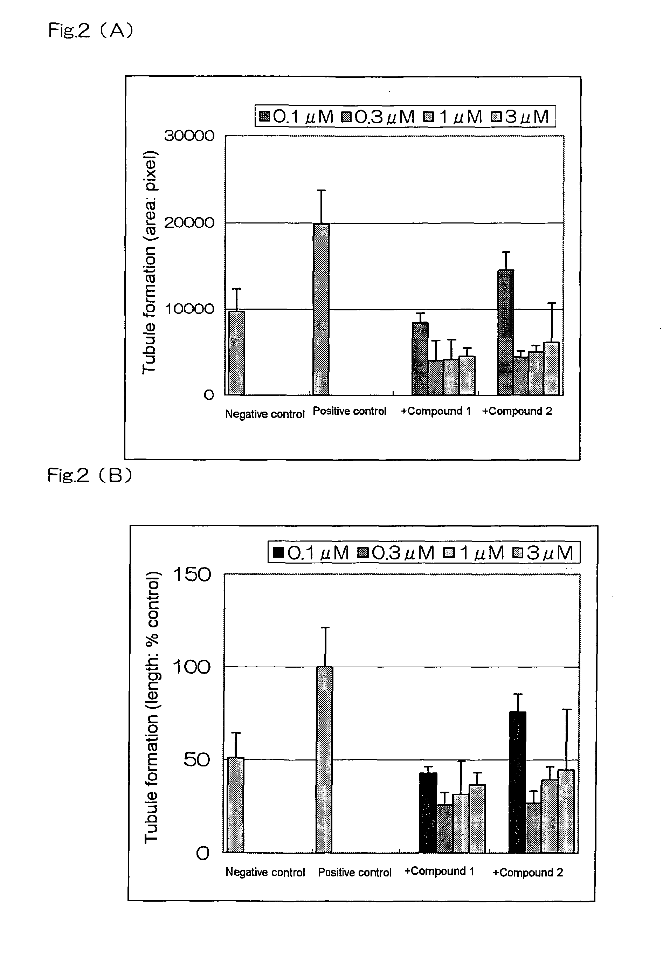

Microscopic images by which the effects of the compounds on angiogenesis were evaluated are shown in Figure 1. Figure 2 is a pair of graphs showing the tubule area (A) and tubule length (B) quantified on the basis of each image data. Five visual fields for each group were photographed and quantified to calculate the mean ± standard error. As shown in Figures 1 and 2, it was demonstrated that compounds 1 and 2 inhibited angiogenesis at concentrations of 0.3 µmol/L or more.

## Claims

1. An angiogenesis inhibitor comprising, as an active ingredient, a cyclic amine compound represented by the general formula (1) : 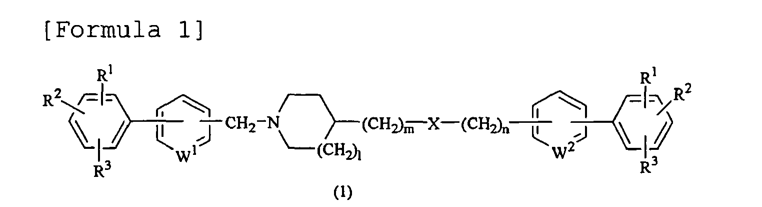 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, or a salt thereof, or a solvate thereof.

2. The angiogenesis inhibitor according to claim 1, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

3. The angiogenesis inhibitor according to claim 1, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

4. The angiogenesis inhibitor according to claim 3, wherein the aryl group, the aryl group of the aralkyl group, the heteroaryl group, or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

5. The angiogenesis inhibitor according to claim 1, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

6. A prophylactic or therapeutic agent against a disease or condition caused by angiogenesis, comprising, as an active ingredient, a cyclic amine compound represented by the general formula (1): 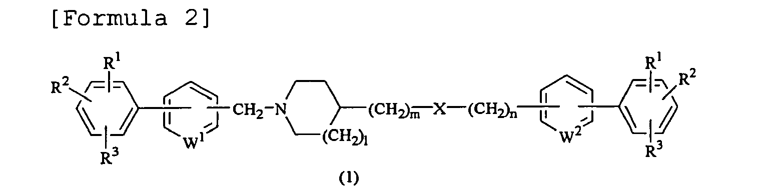 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, or a salt thereof, or a solvate thereof.

7. The prophylactic or therapeutic agent according to claim 6, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

8. The prophylactic or therapeutic agent according to claim 6, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, asubstitutedorunsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

9. The prophylactic or therapeutic agent according to claim 8, wherein the aryl group, the aryl group of the aralkyl group, the heteroaryl group, or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

10. The prophylactic or therapeutic agent according to claim 6, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

11. The prophylactic or therapeutic agent according to claim 6, wherein the disease or pathological condition is proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

12. Use, for producing an angiogenesis inhibitor, of a cyclic amine compound represented by the general formula (1): 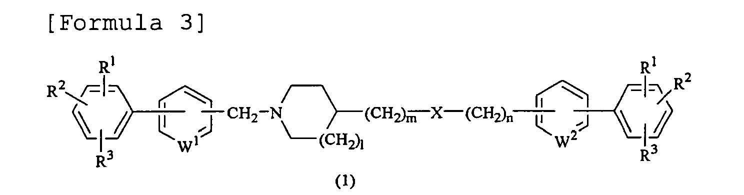 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, or a salt thereof, or a solvate thereof.

13. The use according to claim 12, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

14. The use according to claim 12, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

15. The use according to claim 14, wherein the aryl group, the aryl group of the aralkyl group, the heteroaryl group, or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

16. The use according to claim 12, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

17. Use, for producing a prophylactic or therapeutic agent against a disease or pathological condition caused by angiogenesis, of a cyclic amine compound represented by the general formula (1): 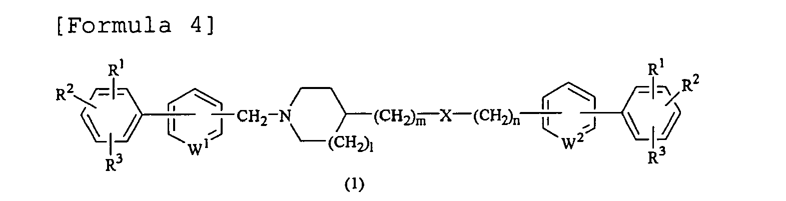 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, or a salt thereof, or a solvate thereof.

18. The use according to claim 17, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

19. The use according to claim 17, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

20. The use according to claim 19, wherein, the aryl group, the aryl group of the aralkyl group, the heteroaryl group, or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

21. The use according to claim 17, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

22. The use according to claim 17, wherein the disease or pathological condition is proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.

23. A method for inhibiting angiogenesis, comprising administering an effective amount of a cyclic amine compound represented by the general formula (1): 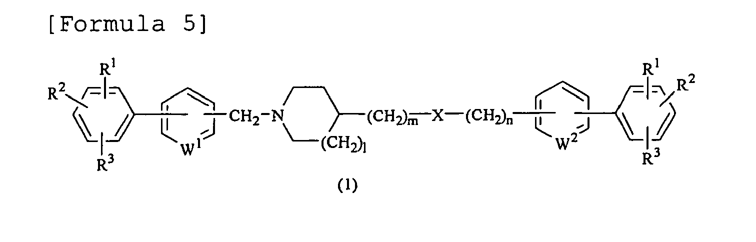 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, or a salt thereof, or a solvate thereof to patients in need thereof.

24. The method according to claim 23, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

25. The method according to claim 23, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, a substituted or unsubstituted 5- or 6-memberedheteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

26. The method according to claim 25, wherein the aryl group, the aryl group of the aralkyl group, the heteroaryl group or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

27. The method according to claim 23, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

28. A method for treating a disease or pathological condition caused by angiogenes is, comprising administer ing an effective amount of a cyclic amine compound represented by the general formula (1): 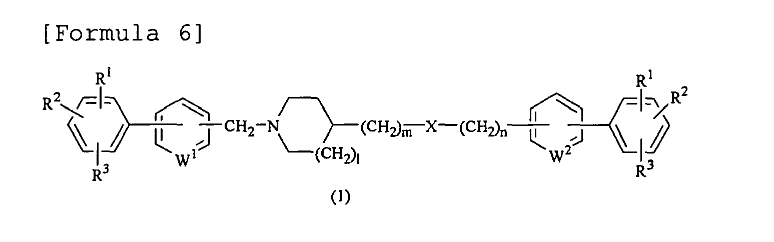 wherein R¹, R², and R³ each independently represent a hydrogen atom, a halogen atom, a hydroxy group, an alkyl group, a halogen-substituted alkyl group, an alkoxy group, an alkylthio group, a carboxyl group, an alkoxycarbonyl group, or an alkanoyl group; W¹ and W² each independently represent N or CH; X represents O, NR⁴, CONR⁴, or NR⁴CO; R⁴ represents a hydrogen atom, an alkyl group, an alkenyl group, an alkynyl group, a substituted or unsubstituted aryl group, a substituted or unsubstituted heteroaryl group, a substituted or unsubstituted aralkyl group, or a substituted or unsubstituted heteroaralkyl group; and 1, m, and n each represent a number of 0 or 1, a salt thereof, or a solvate thereof.

29. The method according to claim 28, wherein R¹, R², and R³ are each a hydrogen atom, a halogen atom, a hydroxy group, a C₁-C₈ alkyl group, a halogen-substituted C₁-C₈ alkyl group, an alkoxy group having a C₁-C₈ alkyl group, an alkylthio group having a C₁-C₈ alkyl group, a carboxyl group, an alkoxycarbonyl group having a C₁-C₆ alkyl group, or an alkanoyl group having a C₁-C₆ alkyl group.

30. The method according to claim 28, wherein R⁴ is a hydrogen atom, a C₁-C₈ alkyl group, a C₃-C₈ alkenyl group, a C₃-C₈ alkynyl group, a substituted or unsubstituted C₆-C₁₄ aryl group, a substituted or unsubstituted 5- or 6-membered heteroaryl group containing 1 to 4 nitrogen atoms, a substituted or unsubstituted C₆-C₁₄ aryl-C₁-C₆ alkyl group, or a C₁-C₆ alkyl group having a substituted or unsubstituted 5- or 6-membered heteroaryl group containing 1 to 4 nitrogen atoms.

31. The method according to claim 30, wherein the aryl group, the aryl group of the aralkyl group, the heteroaryl group or the heteroaryl group of the heteroaralkyl group in R⁴ is substituted with 1 to 3 substituents selected from an alkyl group, an alkoxy group, an alkylthio group, a halogen atom, a nitro group, an amino group, an acetylamino group, a trifluoromethyl group, and an alkylenedioxy group.

32. The method according to claim 28, wherein the active ingredient is 4-[N-(4-methoxyphenyl)-N-[[5-(3,4,5-trimethoxyphenyl)pyr idin-3-yl]methyl]amino]-1-[[2-(3,4,5-trimethoxyphenyl)py ridin-4-yl]methyl]piperidine, 4-[N-(4-methoxyphenyl)-N-[[2-(3,4,5-trimethoxyphenyl)pyr idin-4-yl]methyl]amino]-1-[[2-(3,4,5-trimethoxyphenyl)py ridin-4-yl]methyl]piperidine, or a salt thereof.

33. The method according to claim 28, wherein the disease or pathological condition is proliferation, recurrence, or metastasis of malignant solid tumor, corneal angiogenesis, pterygium, conjunctivitis, rubeosis iridis, neovascular glaucoma, proliferative retinopathy, central retinal vein occlusion, diabetic retinopathy, retinal angiogenesis, or age-related macular degeneration.
